# EUROPEAN PATENT SPECIFICATION

(11) **EP 3 193 922 B1**
(45) Date of publication and mention of the grant of the patent: **28.08.2019**
(21) Application number: 15763360.3
(22) Date of filing: 17.09.2015
(51) Int. Cl.: A61K 39/125

(54) **VACCINE**
IMPFSTOFF
VACCIN

(30) Priority: 18.09.2014 US 201462052145 P
(43) Date of publication of application: 26.07.2017
(73) Proprietor: GlaxoSmithKline Biologicals S.A., 1330 Rixensart (BE)
(72) Inventor: CASTADO, Cindy, B-1330 Rixensart (BE); LABBE, Steve, Laval, Quebec G1P 4R8 (CA); RHEAULT, Patrick, Laval, Quebec G1P 4R8 (CA)
(74) Representative: Thornley, Rachel Mary
(86) International application number: PCT/EP2015/071288
(87) International publication number: WO 2016/042059

(56) References cited:
- WO-A1-97/22701
- WO-A2-2009/090594
- WO-A2-2012/171026
- MALLIA PATRICK ET AL: "Experimental Rhinovirus Infection as a Human Model of Chronic Obstructive Pulmonary Disease Exacerbation", AMERICAN JOURNAL OF RESPIRATORY AND CRITICAL CARE MEDICINE, vol. 183, no. 6, March 2011 (2011-03), pages 734-742, XP002750640, DOI: 10.1164/RCCM.201006-0833OC
- John C Perkins: "EVIDENCE FOR PROTECTIVE EFFECT OF AN INACTIVATED RHINOVIRUS VACCINE ADMINISTERED BY THE NASAL ROUTE", Lab. Infectious Diseases, 1 January 1969 (1969-01-01), pages 319-326, XP055226798, Retrieved from the Internet: URL:http://aje.oxfordjournals.org/content/ 90/4/319.full.pdf [retrieved on 2015-11-09]
- GRGACIC E V L ET AL: "Virus-like particles: Passport to immune recognition", METHODS, ACADEMIC PRESS, US, vol. 40, no. 1, 1 September 2006 (2006-09-01), pages 60-65, XP024908472, ISSN: 1046-2023, DOI: 10.1016/J.YMETH.2006.07.018 [retrieved on 2006-09-01]

### SEQUENCE LISTING

<110> GlaxoSmithKline Biologicals SA
<120> Vaccine
<130> VB65763
<160> 18
<170> PatentIn version 3.5
<210> 1
   <211> 1320
   <212> DNA
   <213> Human rhinovirus
<400> 1
   atgggtcatc accatcatca tcacgggtcc ctgcaggact cagaagtcaa tcaagaagct      60
   aagccagagg tcaagccaga agtcaagcct gagactcaca tcaatttaaa ggtgtccgat     120
   ggatcttcag agatcttctt caagatcaaa aagaccactc ctttaagaag gctgatggaa     180
   gcgttcgcta aaagacaggg taaggaaatg gactccttaa cgttcttgta cgacggtatt     240
   gaaattcaag ctgatcagac ccctgaagat ttggacatgg aggataacga tattattgag     300
   gctcacagag aacagattgg aggtggcgcc caggtgagca cccagaagag cggcagccac     360
   gagaaccaga acatcctgac caacggcagc aaccagacct tcaccgtgat caactactac     420
   aaggacgccg ccagcaccag cagcgccggc cagagcctga gcatggaccc cagcaagttc     480
   accgagcccg tgaaggacct gatgctgaag ggcgcccccg ccctgaacag ccccaacgtg     540
   gaggcctgcg gctacagcga ccgggtgcag cagatcaccc tgggcaacag caccatcacc     600
   acccaggagg ccgccaacgc cgtggtgtgc tacgccgagt ggcccgagta cctgcccgac     660
   gtggacgcca gcgacgtgaa caagaccagc aagcccgaca ccagcgtgtg ccggttctac     720
   accctggaca gcaagacctg gaccaccggc agcaagggct ggtgctggaa gctgcccgac     780
   gccctgaagg acatgggcgt gttcggccag aacatgttct tccacagcct gggccggagc     840
   ggctacaccg tgcacgtgca gtgcaacgcc accaagttcc acagcggctg cctgctggtg     900
   gtggtgatcc ccgagcacca gctggccagc cacgagggcg gcaacgtgag cgtgaagtac     960
   accttcaccc accccggcga gcggggcatc gacctgagca gcgccaacga ggtgggcggc    1020
   cccgtgaagg acgtgatcta caacatgaac ggcaccctgc tgggcaacct gctgatcttc    1080
   ccccaccagt tcatcaacct gcggaccaac aacaccgcca ccatcgtgat cccctacatc    1140
   aacagcgtgc ccatcgacag catgacccgg cacaacaacg tgagcctgat ggtgatcccc    1200
   atcgcccccc tgaccgtgcc caccggcgcc acccccagcc tgcccatcac cgtgaccatc    1260
   gcccccatgt gcaccgagtt cagcggcatc cggagcaaga gcatcgtgcc ccagtgataa    1320
<210> 2
   <211> 438
   <212> PRT
   <213> Human rhinovirus
<400> 2
   Met Gly His His His His His His Gly Ser Leu Gln Asp Ser Glu Val
   1               5                   10                  15
   Asn Gln Glu Ala Lys Pro Glu Val Lys Pro Glu Val Lys Pro Glu Thr
               20                  25                  30
   His Ile Asn Leu Lys Val Ser Asp Gly Ser Ser Glu Ile Phe Phe Lys
           35                  40                  45
   Ile Lys Lys Thr Thr Pro Leu Arg Arg Leu Met Glu Ala Phe Ala Lys
       50                  55                  60
   Arg Gln Gly Lys Glu Met Asp Ser Leu Thr Phe Leu Tyr Asp Gly Ile
   65                  70                  75                  80
   Glu Ile Gln Ala Asp Gln Thr Pro Glu Asp Leu Asp Met Glu Asp Asn
                   85                  90                  95
   Asp Ile Ile Glu Ala His Arg Glu Gln Ile Gly Gly Gly Ala Gln Val
               100                 105                 110
   Ser Thr Gln Lys Ser Gly Ser His Glu Asn Gln Asn Ile Leu Thr Asn
           115                 120                 125
   Gly Ser Asn Gln Thr Phe Thr Val Ile Asn Tyr Tyr Lys Asp Ala Ala
       130                 135                 140
   Ser Thr Ser Ser Ala Gly Gln Ser Leu Ser Met Asp Pro Ser Lys Phe
   145                 150                 155                 160
   Thr Glu Pro Val Lys Asp Leu Met Leu Lys Gly Ala Pro Ala Leu Asn
                   165                 170                 175
   Ser Pro Asn Val Glu Ala Cys Gly Tyr Ser Asp Arg Val Gln Gln Ile
               180                 185                 190
   Thr Leu Gly Asn Ser Thr Ile Thr Thr Gln Glu Ala Ala Asn Ala Val
           195                 200                 205
   Val Cys Tyr Ala Glu Trp Pro Glu Tyr Leu Pro Asp Val Asp Ala Ser
       210                 215                 220
   Asp Val Asn Lys Thr Ser Lys Pro Asp Thr Ser Val Cys Arg Phe Tyr
   225                 230                 235                 240
   Thr Leu Asp Ser Lys Thr Trp Thr Thr Gly Ser Lys Gly Trp Cys Trp
                   245                 250                 255
   Lys Leu Pro Asp Ala Leu Lys Asp Met Gly Val Phe Gly Gln Asn Met
               260                 265                 270
   Phe Phe His Ser Leu Gly Arg Ser Gly Tyr Thr Val His Val Gln Cys
           275                 280                 285
   Asn Ala Thr Lys Phe His Ser Gly Cys Leu Leu Val Val Val Ile Pro
       290                 295                 300
   Glu His Gln Leu Ala Ser His Glu Gly Gly Asn Val Ser Val Lys Tyr
   305                 310                 315                 320
   Thr Phe Thr His Pro Gly Glu Arg Gly Ile Asp Leu Ser Ser Ala Asn
                   325                 330                 335
   Glu Val Gly Gly Pro Val Lys Asp Val Ile Tyr Asn Met Asn Gly Thr
               340                 345                 350
   Leu Leu Gly Asn Leu Leu Ile Phe Pro His Gln Phe Ile Asn Leu Arg
           355                 360                 365
   Thr Asn Asn Thr Ala Thr Ile Val Ile Pro Tyr Ile Asn Ser Val Pro
       370                 375                 380
   Ile Asp Ser Met Thr Arg His Asn Asn Val Ser Leu Met Val Ile Pro
   385                 390                 395                 400
   Ile Ala Pro Leu Thr Val Pro Thr Gly Ala Thr Pro Ser Leu Pro Ile
                   405                 410                 415
   Thr Val Thr Ile Ala Pro Met Cys Thr Glu Phe Ser Gly Ile Arg Ser
               420                 425                 430
   Lys Ser Ile Val Pro Gln
           435
<210> 3
   <211> 1038
   <212> DNA
   <213> Human rhinovirus
<400> 3
   atgggtcatc accatcatca tcacgggtcc ctgcaggact cagaagtcaa tcaagaagct      60
   aagccagagg tcaagccaga agtcaagcct gagactcaca tcaatttaaa ggtgtccgat     120
   ggatcttcag agatcttctt caagatcaaa aagaccactc ctttaagaag gctgatggaa     180
   gcgttcgcta aaagacaggg taaggaaatg gactccttaa cgttcttgta cgacggtatt     240
   gaaattcaag ctgatcagac ccctgaagat ttggacatgg aggataacga tattattgag     300
   gctcacagag aacagattgg aggtggcctg cccaccacca ccctgcccgg cagcggccag     360
   ttcctgacca ccgacgaccg gcagagcccc agcgccctgc ccaactacga gcccaccccc     420
   cggatacaca tccccggcaa ggtgcacaac ctgctggaga tcatccaggt ggacaccctg     480
   atccccatga acaacaccca caccaaggac gaggtgaaca gctacctgat ccccctgaac     540
   gccaaccggc agaacgagca ggtgttcggc accaacctgt tcatcggcga cggcgtgttc     600
   aagaccaccc tgctgggcga gatcgtgcag tactacaccc actggagcgg cagcctgcgg     660
   ttcagcctga tgtacaccgg ccccgccctg agcagcgcca agctgatcct ggcctacacc     720
   ccccccggcg cccggggccc ccaggaccgg cgggaggcca tgctgggcac ccacgtggtg     780
   tgggacatcg gcctgcagag caccatcgtg atgaccatcc cctggaccag cggcgtgcag     840
   ttccggtaca ccgaccccga cacctacacc agcgccggct tcctgagctg ctggtatcag     900
   accagcctga tcctgccccc cgagaccacc ggccaggtgt acctgctgag cttcatcagc     960
   gcctgccccg acttcaagct gcggctgatg aaggacaccc agaccatcag ccagaccgtg    1020
   gccctgaccg agtgataa                                                  1038
<210> 4
   <211> 344
   <212> PRT
   <213> Human rhinovirus
<400> 4
   Met Gly His His His His His His Gly Ser Leu Gln Asp Ser Glu Val
   1               5                   10                  15
   Asn Gln Glu Ala Lys Pro Glu Val Lys Pro Glu Val Lys Pro Glu Thr
               20                  25                  30
   His Ile Asn Leu Lys Val Ser Asp Gly Ser Ser Glu Ile Phe Phe Lys
           35                  40                  45
   Ile Lys Lys Thr Thr Pro Leu Arg Arg Leu Met Glu Ala Phe Ala Lys
       50                  55                  60
   Arg Gln Gly Lys Glu Met Asp Ser Leu Thr Phe Leu Tyr Asp Gly Ile
   65                  70                  75                  80
   Glu Ile Gln Ala Asp Gln Thr Pro Glu Asp Leu Asp Met Glu Asp Asn
                   85                  90                  95
   Asp Ile Ile Glu Ala His Arg Glu Gln Ile Gly Gly Gly Leu Pro Thr
               100                 105                 110
   Thr Thr Leu Pro Gly Ser Gly Gln Phe Leu Thr Thr Asp Asp Arg Gln
           115                 120                 125
   Ser Pro Ser Ala Leu Pro Asn Tyr Glu Pro Thr Pro Arg Ile His Ile
       130                 135                 140
   Pro Gly Lys Val His Asn Leu Leu Glu Ile Ile Gln Val Asp Thr Leu
   145                 150                 155                 160
   Ile Pro Met Asn Asn Thr His Thr Lys Asp Glu Val Asn Ser Tyr Leu
                   165                 170                 175
   Ile Pro Leu Asn Ala Asn Arg Gln Asn Glu Gln Val Phe Gly Thr Asn
               180                 185                 190
   Leu Phe Ile Gly Asp Gly Val Phe Lys Thr Thr Leu Leu Gly Glu Ile
           195                 200                 205
   Val Gln Tyr Tyr Thr His Trp Ser Gly Ser Leu Arg Phe Ser Leu Met
       210                 215                 220
   Tyr Thr Gly Pro Ala Leu Ser Ser Ala Lys Leu Ile Leu Ala Tyr Thr
   225                 230                 235                 240
   Pro Pro Gly Ala Arg Gly Pro Gln Asp Arg Arg Glu Ala Met Leu Gly
                   245                 250                 255
   Thr His Val Val Trp Asp Ile Gly Leu Gln Ser Thr Ile Val Met Thr
               260                 265                 270
   Ile Pro Trp Thr Ser Gly Val Gln Phe Arg Tyr Thr Asp Pro Asp Thr
           275                 280                 285
   Tyr Thr Ser Ala Gly Phe Leu Ser Cys Trp Tyr Gln Thr Ser Leu Ile
       290                 295                 300
   Leu Pro Pro Glu Thr Thr Gly Gln Val Tyr Leu Leu Ser Phe Ile Ser
   305                 310                 315                 320
   Ala Cys Pro Asp Phe Lys Leu Arg Leu Met Lys Asp Thr Gln Thr Ile
                   325                 330                 335
   Ser Gln Thr Val Ala Leu Thr Glu
   340
<210> 5
   <211> 1197
   <212> DNA
   <213> Human rhinovirus
<400> 5
   atgggtcatc accatcatca tcacgggtcc ctgcaggact cagaagtcaa tcaagaagct      60
   aagccagagg tcaagccaga agtcaagcct gagactcaca tcaatttaaa ggtgtccgat     120
   ggatcttcag agatcttctt caagatcaaa aagaccactc ctttaagaag gctgatggaa     180
   gcgttcgcta aaagacaggg taaggaaatg gactccttaa cgttcttgta cgacggtatt     240
   gaaattcaag ctgatcagac ccctgaagat ttggacatgg aggataacga tattattgag     300
   gctcacagag aacagattgg aggtggcctg ggcgacgagc tggaggaggt gatcgtggag     360
   aagaccaagc agaccgtggc cagcatcagc agcggcccca agcacaccca gaaggtgccc     420
   atcctgaccg ccaacgagac cggcgccacc atgcccgtgc tgcccagcga cagcatcgag     480
   acccggacca cctacatgca cttcaacggc agcgagaccg acgtggagtg cttcctgggc     540
   cgggccgcct gcgtgcacgt gaccgagatc cagaacaagg acgccaccgg catcgacaac     600
   caccgggagg ccaagctgtt caacgactgg aagatcaacc tgagcagcct ggtgcagctg     660
   cggaagaagc tggagctgtt cacctacgtg cggttcgaca gcgagtacac catcctggcc     720
   accgccagcc agcccgacag cgccaactac agcagcaacc tggtggtgca ggccatgtac     780
   gtgccccccg gcgcccccaa ccccaaggag tgggacgact acacctggca gagcgccagc     840
   aaccccagcg tgttcttcaa ggtgggcgac accagccggt tcagcgtgcc ctacgtgggc     900
   ctggccagcg cctacaactg cttctacgac ggctacagcc acgacgacgc cgagacccag     960
   tacggcatca ccgtgctgaa ccacatgggc agcatggcct tccggatcgt gaacgagcac    1020
   gacgagcaca agaccctggt gaagatccgg gtgtaccacc gggccaagca cgtggaggcc    1080
   tggattcccc gggccccccg ggccctgccc tacaccagca tcggccggac caactacccc     1140
   aagaacaccg agcccgtgat caagaagcgg aagggcgaca tcaagagcta ctgataa       1197
<210> 6
   <211> 397
   <212> PRT
   <213> Human rhinovirus
<400> 6
   Met Gly His His His His His His Gly Ser Leu Gln Asp Ser Glu Val
   1 5 10 15
   Asn Gln Glu Ala Lys Pro Glu Val Lys Pro Glu Val Lys Pro Glu Thr
               20                  25                  30
   His Ile Asn Leu Lys Val Ser Asp Gly Ser Ser Glu Ile Phe Phe Lys
           35                  40                  45
   Ile Lys Lys Thr Thr Pro Leu Arg Arg Leu Met Glu Ala Phe Ala Lys
       50                  55                  60
   Arg Gln Gly Lys Glu Met Asp Ser Leu Thr Phe Leu Tyr Asp Gly Ile
   65                  70                  75                  80
   Glu Ile Gln Ala Asp Gln Thr Pro Glu Asp Leu Asp Met Glu Asp Asn
                   85                  90                  95
   Asp Ile Ile Glu Ala His Arg Glu Gln Ile Gly Gly Gly Leu Gly Asp
               100                 105                 110
   Glu Leu Glu Glu Val Ile Val Glu Lys Thr Lys Gln Thr Val Ala Ser
           115                 120                 125
   Ile Ser Ser Gly Pro Lys His Thr Gln Lys Val Pro Ile Leu Thr Ala
       130                 135                 140
   Asn Glu Thr Gly Ala Thr Met Pro Val Leu Pro Ser Asp Ser Ile Glu
   145                 150                 155                 160
   Thr Arg Thr Thr Tyr Met His Phe Asn Gly Ser Glu Thr Asp Val Glu
                   165                 170                 175
   Cys Phe Leu Gly Arg Ala Ala Cys Val His Val Thr Glu Ile Gln Asn
               180                 185                 190
   Lys Asp Ala Thr Gly Ile Asp Asn His Arg Glu Ala Lys Leu Phe Asn
           195                 200                 205
   Asp Trp Lys Ile Asn Leu Ser Ser Leu Val Gln Leu Arg Lys Lys Leu
       210                 215                 220
   Glu Leu Phe Thr Tyr Val Arg Phe Asp Ser Glu Tyr Thr Ile Leu Ala
   225                 230                 235                 240
   Thr Ala Ser Gln Pro Asp Ser Ala Asn Tyr Ser Ser Asn Leu Val Val
                   245                 250                 255
   Gln Ala Met Tyr Val Pro Pro Gly Ala Pro Asn Pro Lys Glu Trp Asp
               260                 265                 270
   Asp Tyr Thr Trp Gln Ser Ala Ser Asn Pro Ser Val Phe Phe Lys Val
           275                 280                 285
   Gly Asp Thr Ser Arg Phe Ser Val Pro Tyr Val Gly Leu Ala Ser Ala
       290                 295                 300
   Tyr Asn Cys Phe Tyr Asp Gly Tyr Ser His Asp Asp Ala Glu Thr Gln
   305                 310                 315                 320
   Tyr Gly Ile Thr Val Leu Asn His Met Gly Ser Met Ala Phe Arg Ile
                   325                 330                 335
   Val Asn Glu His Asp Glu His Lys Thr Leu Val Lys Ile Arg Val Tyr
               340                 345                 350
   His Arg Ala Lys His Val Glu Ala Trp Ile Pro Arg Ala Pro Arg Ala
           355                 360                 365
   Leu Pro Tyr Thr Ser Ile Gly Arg Thr Asn Tyr Pro Lys Asn Thr Glu
       370                 375                 380
   Pro Val Ile Lys Lys Arg Lys Gly Asp Ile Lys Ser Tyr
   385                 390                 395
<210> 7
   <211> 14
   <212> PRT
   <213> Human rhinovirus
<400> 7
   Pro Ile Leu Thr Ala Asn Glu Thr Gly Ala Thr Met Pro Val
   1               5                   10
<210> 8
   <211> 14
   <212> PRT
   <213> Human rhinovirus
<400> 8
   Pro Ala Leu Asp Ala Ala Glu Thr Gly His Thr Ser Ser Val
   1               5                   10
<210> 9
   <211> 14
   <212> PRT
   <213> Human rhinovirus
<400> 9
   Pro Ile Leu Asp Ala Ala Glu Thr Gly His Thr Ser Asn Val
   1 5 10
<210> 10
   <211> 14
   <212> PRT
   <213> Human rhinovirus
<400> 10
   Gln Ala Leu Gly Ala Val Glu Ile Gly Ala Thr Ala Asp Val
   1 5 10
<210> 11
   <211> 16
   <212> PRT
   <213> Human rhinovirus
<400> 11
   Gly Ala Gln Val Ser Thr Gln Lys Ser Gly Ser His Glu Asn Gln Asn
   1 5 10 15
<210> 12
   <211> 16
   <212> PRT
   <213> Human rhinovirus
<400> 12
   Gly Ala Gln Val Ser Arg Gln Asn Val Gly Thr His Ser Thr Gln Asn
   1 5 10 15
<210> 13
   <211> 16
   <212> PRT
   <213> Human rhinovirus
<400> 13
   Gly Ala Gln Val Ser Arg Gln Ser Val Gly Ser His Glu Thr Met Ile
   1 5 10 15
<210> 14
   <211> 330
   <212> PRT
   <213> Human rhinovirus
<400> 14
   Gly Ala Gln Val Ser Thr Gln Lys Ser Gly Ser His Glu Asn Gln Asn
   1               5                   10                  15
   Ile Leu Thr Asn Gly Ser Asn Gln Thr Phe Thr Val Ile Asn Tyr Tyr
               20                  25                  30
   Lys Asp Ala Ala Ser Thr Ser Ser Ala Gly Gln Ser Leu Ser Met Asp
           35                  40                  45
   Pro Ser Lys Phe Thr Glu Pro Val Lys Asp Leu Met Leu Lys Gly Ala
       50                  55                  60
   Pro Ala Leu Asn Ser Pro Asn Val Glu Ala Cys Gly Tyr Ser Asp Arg
   65                  70                  75                  80
   Val Gln Gln Ile Thr Leu Gly Asn Ser Thr Ile Thr Thr Gln Glu Ala
                   85                  90                  95
   Ala Asn Ala Val Val Cys Tyr Ala Glu Trp Pro Glu Tyr Leu Pro Asp
               100                 105                 110
   Val Asp Ala Ser Asp Val Asn Lys Thr Ser Lys Pro Asp Thr Ser Val
           115                 120                 125
   Cys Arg Phe Tyr Thr Leu Asp Ser Lys Thr Trp Thr Thr Gly Ser Lys
       130                 135                 140
   Gly Trp Cys Trp Lys Leu Pro Asp Ala Leu Lys Asp Met Gly Val Phe
   145                 150                 155                 160
   Gly Gln Asn Met Phe Phe His Ser Leu Gly Arg Ser Gly Tyr Thr Val
                   165                 170                 175
   His Val Gln Cys Asn Ala Thr Lys Phe His Ser Gly Cys Leu Leu Val
               180                 185                 190
   Val Val Ile Pro Glu His Gln Leu Ala Ser His Glu Gly Gly Asn Val
           195                 200                 205
   Ser Val Lys Tyr Thr Phe Thr His Pro Gly Glu Arg Gly Ile Asp Leu
       210                 215                 220
   Ser Ser Ala Asn Glu Val Gly Gly Pro Val Lys Asp Val Ile Tyr Asn
   225                 230                 235                 240
   Met Asn Gly Thr Leu Leu Gly Asn Leu Leu Ile Phe Pro His Gln Phe
                   245                 250                 255
   Ile Asn Leu Arg Thr Asn Asn Thr Ala Thr Ile Val Ile Pro Tyr Ile
               260                 265                 270
   Asn Ser Val Pro Ile Asp Ser Met Thr Arg His Asn Asn Val Ser Leu
           275                 280                 285
   Met Val Ile Pro Ile Ala Pro Leu Thr Val Pro Thr Gly Ala Thr Pro
       290                 295                 300
   Ser Leu Pro Ile Thr Val Thr Ile Ala Pro Met Cys Thr Glu Phe Ser
   305                 310                 315                 320
   Gly Ile Arg Ser Lys Ser Ile Val Pro Gln
                   325                 330
<210> 15
   <211> 289
   <212> PRT
   <213> Human rhinovirus
<400> 15
   Gly Leu Gly Asp Glu Leu Glu Glu Val Ile Val Glu Lys Thr Lys Gln
   1               5                   10                  15
   Thr Val Ala Ser Ile Ser Ser Gly Pro Lys His Thr Gln Lys Val Pro
               20                  25                  30
   Ile Leu Thr Ala Asn Glu Thr Gly Ala Thr Met Pro Val Leu Pro Ser
           35                  40                  45
   Asp Ser Ile Glu Thr Arg Thr Thr Tyr Met His Phe Asn Gly Ser Glu
       50                  55                  60
   Thr Asp Val Glu Cys Phe Leu Gly Arg Ala Ala Cys Val His Val Thr
   65                  70                  75                  80
   Glu Ile Gln Asn Lys Asp Ala Thr Gly Ile Asp Asn His Arg Glu Ala
                   85                  90                  95
   Lys Leu Phe Asn Asp Trp Lys Ile Asn Leu Ser Ser Leu Val Gln Leu
               100                 105                 110
   Arg Lys Lys Leu Glu Leu Phe Thr Tyr Val Arg Phe Asp Ser Glu Tyr
           115                 120                 125
   Thr Ile Leu Ala Thr Ala Ser Gln Pro Asp Ser Ala Asn Tyr Ser Ser
       130                 135                 140
   Asn Leu Val Val Gln Ala Met Tyr Val Pro Pro Gly Ala Pro Asn Pro
   145                 150                 155                 160
   Lys Glu Trp Asp Asp Tyr Thr Trp Gln Ser Ala Ser Asn Pro Ser Val
                   165                 170                 175
   Phe Phe Lys Val Gly Asp Thr Ser Arg Phe Ser Val Pro Tyr Val Gly
   180 185 190
   Leu Ala Ser Ala Tyr Asn Cys Phe Tyr Asp Gly Tyr Ser His Asp Asp
   195 200 205
   Ala Glu Thr Gln Tyr Gly Ile Thr Val Leu Asn His Met Gly Ser Met
   210 215 220
   Ala Phe Arg Ile Val Asn Glu His Asp Glu His Lys Thr Leu Val Lys
   225 230 235 240
   Ile Arg Val Tyr His Arg Ala Lys His Val Glu Ala Trp Ile Pro Arg
   245 250 255
   Ala Pro Arg Ala Leu Pro Tyr Thr Ser Ile Gly Arg Thr Asn Tyr Pro
   260 265 270
   Lys Asn Thr Glu Pro Val Ile Lys Lys Arg Lys Gly Asp Ile Lys Ser
   275 280 285
   Tyr
<210> 16
   <211> 262
   <212> PRT
   <213> Human rhinovirus
<400> 16
   Ser Pro Asn Val Glu Ala Cys Gly Tyr Ser Asp Arg Val Gln Gln Ile
   1 5 10 15
   Thr Leu Gly Asn Ser Thr Ile Thr Thr Gln Glu Ala Ala Asn Ala Val
   20 25 30
   Val Cys Tyr Ala Glu Trp Pro Glu Tyr Leu Pro Asp Val Asp Ala Ser
   35 40 45
   Asp Val Asn Lys Thr Ser Lys Pro Asp Thr Ser Val Cys Arg Phe Tyr
   50 55 60
   Thr Leu Asp Ser Lys Thr Trp Thr Thr Gly Ser Lys Gly Trp Cys Trp
   65 70 75 80
   Lys Leu Pro Asp Ala Leu Lys Asp Met Gly Val Phe Gly Gln Asn Met
   85 90 95
   Phe Phe His Ser Leu Gly Arg Ser Gly Tyr Thr Val His Val Gln Cys
   100 105 110
   Asn Ala Thr Lys Phe His Ser Gly Cys Leu Leu Val Val Val Ile Pro
   115 120 125
   Glu His Gln Leu Ala Ser His Glu Gly Gly Asn Val Ser Val Lys Tyr
   130 135 140
   Thr Phe Thr His Pro Gly Glu Arg Gly Ile Asp Leu Ser Ser Ala Asn
   145 150 155 160
   Glu Val Gly Gly Pro Val Lys Asp Val Ile Tyr Asn Met Asn Gly Thr
   165 170 175
   Leu Leu Gly Asn Leu Leu Ile Phe Pro His Gln Phe Ile Asn Leu Arg
   180 185 190
   Thr Asn Asn Thr Ala Thr Ile Val Ile Pro Tyr Ile Asn Ser Val Pro
   195 200 205
   Ile Asp Ser Met Thr Arg His Asn Asn Val Ser Leu Met Val Ile Pro
   210 215 220
   Ile Ala Pro Leu Thr Val Pro Thr Gly Ala Thr Pro Ser Leu Pro Ile
   225 230 235 240
   Thr Val Thr Ile Ala Pro Met Cys Thr Glu Phe Ser Gly Ile Arg Ser
   245 250 255
   Lys Ser Ile Val Pro Gln
   260
<210> 17
   <211> 236
   <212> PRT
   <213> Human rhinovirus
<400> 17
   Gly Leu Pro Thr Thr Thr Leu Pro Gly Ser Gly Gln Phe Leu Thr Thr
   1 5 10 15
   Asp Asp Arg Gln Ser Pro Ser Ala Leu Pro Asn Tyr Glu Pro Thr Pro
   20 25 30
   Arg Ile His Ile Pro Gly Lys Val His Asn Leu Leu Glu Ile Ile Gln
   35 40 45
   Val Asp Thr Leu Ile Pro Met Asn Asn Thr His Thr Lys Asp Glu Val
       50                  55                  60
   Asn Ser Tyr Leu Ile Pro Leu Asn Ala Asn Arg Gln Asn Glu Gln Val
   65                  70                  75                  80
   Phe Gly Thr Asn Leu Phe Ile Gly Asp Gly Val Phe Lys Thr Thr Leu
                   85                  90                  95
   Leu Gly Glu Ile Val Gln Tyr Tyr Thr His Trp Ser Gly Ser Leu Arg
               100                 105                 110
   Phe Ser Leu Met Tyr Thr Gly Pro Ala Leu Ser Ser Ala Lys Leu Ile
           115                 120                 125
   Leu Ala Tyr Thr Pro Pro Gly Ala Arg Gly Pro Gln Asp Arg Arg Glu
       130                 135                 140
   Ala Met Leu Gly Thr His Val Val Trp Asp Ile Gly Leu Gln Ser Thr
   145                 150                 155                 160
   Ile Val Met Thr Ile Pro Trp Thr Ser Gly Val Gln Phe Arg Tyr Thr
                   165                 170                 175
   Asp Pro Asp Thr Tyr Thr Ser Ala Gly Phe Leu Ser Cys Trp Tyr Gln
               180                 185                 190
   Thr Ser Leu Ile Leu Pro Pro Glu Thr Thr Gly Gln Val Tyr Leu Leu
           195                 200                 205
   Ser Phe Ile Ser Ala Cys Pro Asp Phe Lys Leu Arg Leu Met Lys Asp
       210                 215                 220
   Thr Gln Thr Ile Ser Gln Thr Val Ala Leu Thr Glu
   225                 230                 235
<210> 18
   <211> 68
   <212> PRT
   <213> Human rhinovirus
<400> 18
   Gly Ala Gln Val Ser Thr Gln Lys Ser Gly Ser His Glu Asn Gln Asn
   1               5                   10                  15
   Ile Leu Thr Asn Gly Ser Asn Gln Thr Phe Thr Val Ile Asn Tyr Tyr
               20                  25                  30
   Lys Asp Ala Ala Ser Thr Ser Ser Ala Gly Gln Ser Leu Ser Met Asp
           35                  40                  45
   Pro Ser Lys Phe Thr Glu Pro Val Lys Asp Leu Met Leu Lys Gly Ala
       50                  55                  60
   Pro Ala Leu Asn
   65

## Description

### BACKGROUND

The present disclosure relates to the field of human vaccines. More particularly, the present disclosure relates to pharmaceutical and immunogenic compositions, for the prevention or treatment of human infection or disease, in particular human rhinovirus (HRV) infection or disease.

Rhinoviruses are non-enveloped viruses and are composed of a capsid formed from four viral proteins VP1, VP2, VP3 and VP4. VP1, VP2, and VP3 form the major part of the protein capsid. The much smaller VP4 protein of approximately 70 amino acids in length has a more extended structure, and lies at the interface between the capsid and the RNA genome. The capsid is composed of 60 copies of each of these proteins assembled as an icosahedron.

The rhinovirus genome consists of a linear, single-stranded, positive sense RNA of between 7.2 and 8.5 kb in length. Structural proteins are encoded in the 5' region of the genome (starting from the 5' end: VP4, VP2, VP3 and VP1) and nonstructural at the 3' end, as is the case for all picornaviruses. The RNA is translated into a single polyprotein that is cleaved co-translationally and post-translationally into the four structural proteins and seven non structural proteins. The non structural genes are involved in processing the viral genome, viral replication, and shutting down the host cell protein production.

Currently there are over 100 HRV serotypes. Based on nucleotide identity and susceptibility of antiviral compounds HRVs have been classified into clades A, B, C and possibly D (Rollinger & Schmidtke, 2011; Palmenberg, Rathe & Liggett, 2010), see Table below.

| **Clades** | **Receptor type** | **Serotypes numbers** | **Serotypes examples** | **Remarks** |
|---|---|---|---|---|
| **HRV-A** | *Major* | 62 | 16 | |
| **HRV-A** | *Minor* | 12 | 1A, 1B, 2, 23, 25, 29, 30, 31, 44, 47, 49, 62 | |
| **HRV-B** | *Major* | 25 | 3, 14 | |
| **HRV-C** | *?* | 7 | | New, emerging, clade |
| **HRV87** | *?* | 1 | | Same virus as Human Enterovirus 68, of species HEV-D (Blomqvist et al., 2002) |
| **(HRV-D)** | *Major* | 3 | 8, 45, 95 | Potentially separated clade (distinct from other clades based on VP3 and nonstructural proteins but not VP1 and VP4) |

In addition host cell receptor specificity has been used to further classify these viruses into major and minor groups. Serotypes that use the intercellular adhesion molecule 1 (ICAM-1) receptor (62 HRV-A serotypes and all the B serotypes) belong to the major receptor group and the remaining 12 HRV-A serotypes use members of the low-density lipoprotein (LDL) receptor family and belong to the minor receptor group. Therefore the terms "HRV-A major", "HRV -A minor", and "HRV-B major" are used.

Serotypes are further classified by the antigenic sites they utilise to evade the host's immune system. For the major receptor group four primary neutralising immunogenic (NIm) sites have been mapped to protruding regions on the external capsid proteins VP1, VP2 and VP3. These are known as NIm-IA, NIm-IB, NIm-II and NIm-III. For the minor receptor serotypes there are three distinct antigenic sites A, B and C that are located in the same vicinity as the NIm sites (reviewed in Lewis-Rogers et al 2009 Mol Biol Evol 26:969).

Rhinoviruses are the primary cause of acute upper respiratory tract infections in humans, known as the common cold. They are also the most common viral cause of severe exacerbation of chronic respiratory diseases such as asthma and chronic obstructive pulmonary disease (COPD).

The provision of a vaccine against HRV is a particular challenge due to the large number of serotypes of the virus and the lack of a protective response generated in individuals infected with one serotype against infection with another serotype.

No vaccine has so far been developed. A rhinovirus vaccine, which would need to be able to protect against multiple serotypes, therefore represents a large unmet medical need.

One approach under evaluation is the provision of peptides which are conserved among HRV serotypes. It has been demonstrated that antibodies induced with recombinant HRV-14 or -89 VP1 proteins or a peptide spanning amino acids 147-162 of HRV14 VP1 exhibit specific and cross-neutralizing activity (McCray & Werner, 1998 Nature 329:736; Edlmayr et al., 2011 Eur. Respir 37:44). Antibodies raised against the N terminal 30 amino acids of VP4 were found to neutralise HRV14, HRV16 and HRV29. In addition, antibodies raised to a consensus sequence of the first 24 residues from rhinovirus VP4 also had some cross-neutralising activity (Katpally et al, 2009 J. Virol 83:7040).

WO 2006/078648 relates to peptide vaccines against HRV derived from the transiently exposed regions of VP4 in particular amino acids 1-31 or 1-24 of VP4; WO 2011/050384 relates to peptides from the N terminus of VP1 including amino acids 1-8; WO 2008/057158 relates to NIm IV of rhinovirus, in particular a peptide comprising amino acids 277-283 or 275-285 from the carboxyl terminal region of VP1, in particular from HRV-14.

Virus-Like Particles (VLPs) are an attractive vehicle for antigen presentation, as they provide antigens in a structural environment similar to the structural environment in the virus, yet are not infectious. However, strategies for producing VLPs or sub-VLPs (protomers, pentamers or else) that have worked for other picornaviruses, such as enterovirus (Chung et al. 2010 Vaccine 28, 6951) and FMDV (Porta et al. 2013 J Virol Methods 187:406) have failed for human rhinoviruses.

### BRIEF SUMMARY

Using a novel method, wherein capsid proteins were expressed as fusion proteins with SUMO sequences, the inventors have now been able to generate rhinoviral VLPs.

Accordingly in a first aspect, the invention relates to a virus-like particle (VLP), comprising human rhinovirus capsid proteins VP0, VP1 and VP3, or, comprising human rhinovirus capsid proteins VP1, VP2, VP3 and VP4.

In a further aspect, the invention relates to an immunogenic composition comprising a VLP according to the invention and a pharmaceutically acceptable diluent, excipient or carrier.

In a further aspect, the invention relates to the VLP or immunogenic composition according to the invention for use in a method for inducing an immune response in a human against rhinovirus.

The present disclosure also relates to the use of an immunogenic composition as described herein in the manufacture of a medicament for the prevention or treatment of rhinoviral infection, such as prevention or treatment of common cold.

The present disclosure also relates to a method for inducing an immune response, such as an immune response involving neutralising antibodies and/or a cellular response, against rhinovirus in humans comprising administering to a human an immunogenic composition as described herein.

The present disclosure also relates to a method for inducing an immune response, such as an immune response involving cross-neutralising antibodies and/or a cellular response, against rhinovirus in humans comprising administering to a human an immunogenic composition as described herein.

The present disclosure also relates to a method for preventing rhinovirus infection or disease related to rhinovirus, which method comprises administering to a human an immunogenic composition as described herein.

The present disclosure also relates to the use of an immunogenic composition as described herein, in the manufacture of a medicament for the prevention or treatment of COPD or asthma exacerbations.

In one embodiment, the invention relates to the VLP or immunogenic composition of the invention wherein the VLP is produced by a method for producing rhinoviral VLPs comprising the steps of:
a. producing constructs encoding capsid proteins required for the formation of a VLP, wherein each of the capsid proteins is encoded as a fusion protein with a chaperone protein, such as SUMO,
b. co-expressing said constructs in the same host cell or expressing one or more of said constructs in separate host cells, and
c. lysing said host cells and bringing, in case of separate expression, all capsid proteins required for a VLP together.

### BRIEF DESCRIPTION OF THE DRAWINGS

Figure 1: Anti-His Western blot showing expression and solubility of individually and co-transfected His-SUMO-VPs.
Figure 2: TEM after negative staining of VLP-VP-SUMO samples.
Figure 3: Coomassie-stained LDS-PAGE showing molecular weight shift after SUMO-protease treatment of semi-purified SUMO-VPs fusion proteins.
Figure 4: Western blot (anti-VPl antibody) showing molecular weight shift before (A) and after (B) SUMO-protease treatment of His-SUMO-VP1 fusion protein.

### DETAILED DESCRIPTION

### DEFINITIONS

The term "virus like particle" (VLP) refers to a viral capsid which resembles the external protein structure of the native virus but is non-infectious because it does not contain viral genetic material. Typically, the size of the VLP is similar to that of the virus and/or its structure is icosahedral, composed of repeated identical protein subunits known as capsomeres.

References to amino acid positions, when used herein, refer to positions in capsid proteins of HRV14 (set forth in SEQ ID NOs:14 to 18), or to structurally equivalent positions in capsid proteins of other HRV serotypes. Such structurally equivalent positions are positions that align with a given position in the most optimal alignment between the other serotype capsid protein sequence and that of the HRV14 sequence. For example, position 33 in a given serotype may be structurally equivalent to position 32 in HRV14 if these positions align in the most optimal alignment of the two protein sequences.

The terms "modify" or "modification" where used herein in connection with a capsid protein indicate a modification of the protein as compared to the sequence of that protein found in nature. A modification is preferably a deletion, substitution or insertion of an amino acid, i.e. deletion, substitution or insertion of one or more amino acid. However, the modification may alternatively be a post-translational modification, such as a modification masking a region of the protein. It will be understood, that in the context of this disclosure, there are numerous naturally occurring serotypes of HRV (and HRV proteins), e.g., obtained from different naturally occurring serotypes of HRV.

"Capsid proteins" of a rhinovirus include VP0, VP1, VP2, VP3 and VP4.

The term "human rhinovirus" abbreviated to HRV refers to any serotype of rhinovirus in the family *Picornaviridae* which is capable of infecting humans and has been identified or has yet to be identified as a rhinovirus. There are several different ways of grouping HRVs as described herein, and each grouping contains multiple virus "serotypes" or "strains" (e.g., HRV-14, HRV-8, HRV-25, etc.) categorized by genetic similarity.

A "variant" when referring to a nucleic acid or a protein (e.g., a VP1 or VP4 nucleic acid or polypeptide) is a nucleic acid or a protein that differs from a reference nucleic acid or protein. Usually, the difference(s) between the variant and the reference nucleic acid or polypeptide constitute a proportionally small number of differences as compared to the referent. A variant protein can differ from the reference protein to which it is compared by the addition, deletion or substitution of one or more amino acids, or by the substitution of an amino acid analogue.

As used herein, a first amino acid sequence has "x% identity" to a second amino acid sequence means that x% represents the number of amino acids in the first sequence which are identical to their matched amino acids of the second sequence when both sequences are optimally aligned, relative to the total length of the second amino acid sequence. Both sequences are optimally aligned when x is maximum. The alignment and the determination of the percentage of identity may be carried out manually or automatically using BLAST.

The term "epitope" refers to a site on an antigen to which B and/or T cells respond. The "immunodominant epitopes" are those epitopes to which a functionally significant host immune response, e.g., an antibody response or a T-cell response, is primarily made.

An "immunogenic composition" is a composition of matter suitable for administration to a human or animal subject (e.g., in an experimental setting) that is capable of eliciting or inducing a specific immune response, e.g., against a pathogen, such as a rhinovirus. As such, an immunogenic composition includes one or more antigens, for example VLPs as described herein. An immunogenic composition can also include one or more additional components, such as an excipient, carrier, and/or adjuvant. In certain instances, immunogenic compositions are administered to elicit or induce an immune response that protects the subject against symptoms or conditions induced by a pathogen. In the context of this disclosure, the term immunogenic composition will be understood to encompass compositions that are intended for administration to a subject or population of subjects for the purpose of eliciting or inducing a protective or palliative immune response against e.g. HRV (that is, vaccine compositions or vaccines).

An "immune response" is a response of a cell of the immune system, such as a B cell, T cell, or monocyte, to a stimulus. An immune response can be a B cell response, which results in the production of specific antibodies, such as antigen specific neutralizing antibodies. An immune response can also be a T cell response, such as a CD4+ response or a CD8+ response. An immune response is a cross-reactive immune response when it is elicited by an antigen from one serotype and reacts not only with virus from that serotype, but also virus from a different serotype.

"Attenuation" of an epitope, when used herein, refers to modification of an epitope which renders the epitope less immunogenic.

### ASPECTS AND EMBODIMENTS OF THE INVENTION

As described above, in a first aspect, the invention relates to a virus-like particle (VLP) comprising human rhinovirus capsid proteins VP0, VP1 and VP3, or, comprising human rhinovirus capsid proteins VP1, VP2, VP3 and VP4.

The capsid proteins in the VLP may be of one serotype or of different serotypes. Thus, in one embodiment, all capsid proteins originate from the same serotype. In another embodiment, the capsid proteins originate from two or more different serotypes, such as two or more different serotypes selected from the group consisting of: HRV 1B, 2, 3, 8, 10, 14, 26, 29, 31, 39, 47, 61, 62, 63, 66, 77, 97 and 100.

### Protein modifications

The VLP according to the invention may only comprise naturally-occurring capsid proteins, e.g. from a specific serotype mentioned herein. However, in another embodiment, one or more capsid proteins has been modified to make it immunogenically more similar to other serotypes, thus increasing its potential to induce an immune response which will also recognise rhinoviruses of other serotypes.

Thus, in one embodiment, one or more of the capsid proteins comprises at least one modification which increases the ability of the VLP to induce a cross-reactive immune response against multiple serotypes, as compared to a VLP wherein said modification is not present. Such a cross-reactive immune response may involve cross-reactive antibodies and/or cross-reactive cellular responses.

In one embodiment, the modification consists of insertion of a peptide from a rhinoviral capsid protein, wherein said peptide is capable of inducing a cross-reactive immune response against two or more serotypes. Typically, the inserted peptides comprises sequences that are conserved between multiple serotypes. The insertion site for the peptide should be chosen so that they do not prevent the formation of VLPs. Formation of VLPs can be tested as described in the Examples herein.

In a further embodiment hereof, said VLP (i.e. the capsid proteins of the VLP) comprises 2, 3, 4 or more insertions of peptides from rhinoviral capsid proteins, optionally from 2 or more different capsid proteins, e.g. insertion of a peptide from VP1 and insertion of a peptide from VP4. In a further embodiment, one, more or all of said peptides consist of fewer than 50 amino acids of the capsid protein, such as between 10 and 30, e.g. between 8 and 18 amino acids of the capsid protein . In an even further embodiment, one of the peptides is amino acids 32-45 from VP1 or a variant of amino acids 32-45 of VP1 having 1-4 amino acid additions or deletions at either end and/or 1-2 amino acid substitutions or additions or deletions within the peptide sequence. In a specific embodiment, the peptide is selected from:

| | | |
|---|---|---|
| HRV14 (B): | 32-PILTANETGATMPV-45 | [SEQ ID NO: 7] |
| HRV8 (A-M): | 32-PALDAAETGHTSSV-45 | [SEQ ID NO: 8] |
| HRV25(A-m): | 32-PILDAAETGHTSNV-45 | [SEQ ID NO: 9] |
| HRV_C_026: | 32-QALGAVEIGATADV-45 | [SEQ ID NO: 10] |

or a variant thereof having 1-4 amino acid additions or deletions at either end and/or 1-2 amino acid substitutions or additions or deletions within the peptide sequence.

In a further embodiment, one of the peptides is amino acids 1-16 from VP4 or a variant of amino acids 1-16 of VP4 having 1-4 amino acid additions or deletions at either end and/or 1-2 amino acid substitutions or additions or deletions within the peptide sequence. In a specific embodiment, the peptide is selected from:

| | | |
|---|---|---|
| HRV14 (B): | 1-GAQVSTQKSGSHENQN-16 | [SEQ ID NO: 11] |
| HRV100(A-M): | 1-GAQVSRQNVGTHSTQN-16 | [SEQ ID NO: 12] |
| HRV_C_026: | 1-GAQVSRQSVGSHETMI-16 | [SEQ ID NO: 13] |

or a variant thereof having 1-4 amino acid additions or deletions at either end and/or 1-2 amino acid substitutions or additions or deletions within the peptide sequence.

In a further embodiment, one of the peptides is amino acids 147-162 of VP1 (e.g. VP1 of HRV14) or a variant of amino acids 147-162 of VP1 having 1-4 amino acid additions or deletions at either end and/or 1-2 amino acid substitutions or additions or deletions within the peptide sequence.

In a further embodiment, one of the peptides is amino acids 1-30 of VP4 (e.g. VP4 of HRV14) or a variant of amino acids 1-30 of VP4 having 1-4 amino acid additions or deletions at either end and/or 1-2 amino acid substitutions or additions or deletions within the peptide sequence.

In a further embodiment, one of the peptides is amino acids 1-24 of VP4 (e.g. VP4 of HRV14) or a variant of amino acids 1-24 of VP4 having 1-4 amino acid additions or deletions at either end and/or 1-2 amino acid substitutions or additions or deletions within the peptide sequence.

In a further embodiment, one of the peptides is amino acids 1-8 of VP1 (e.g. VP1 of HRV14) or a variant of amino acids 1-8 of VP1 having 1-4 amino acid additions or deletions at either end and/or 1-2 amino acid substitutions or additions or deletions within the peptide sequence.

In a further embodiment, one of the peptides is amino acids 277-283 of VP1 (e.g. VP1 of HRV14) or a variant of amino acids 277-283 of VP1 having 1-4 amino acid additions or deletions at either end and/or 1-2 amino acid substitutions or additions or deletions within the peptide sequence.

In a further embodiment, one of the peptides is amino acids 275-285 of VP1 (e.g. VP1 of HRV14) or a variant of amino acids 275-285 of VP1 having 1-4 amino acid additions or deletions at either end and/or 1-2 amino acid substitutions or additions or deletions within the peptide sequence.

In particular embodiments, amino acid substitutions as referred to herein are conservative substitutions.

Several positions on the capsid protein are suitable for insertion of such peptides.

In one embodiment, the peptide has been inserted in VP0, such as in VP0 of HRV14, e.g. in one or more of the regions corresponding to 72-76, 131-175, 133-145, 158-165, 228-238, 231-237 or 255-262 of VP2.

In a further embodiment, the peptide has been inserted in VP1, such as in VP1 of HRV14, e.g. in one or more of the regions 73-102, 85-92, 129-149, 136-145, 160-168, 204-212, 208-215, 230-236 or 264-289 of VP1.

In a further embodiment, the peptide has been inserted in VP2, such as in VP2 of HRV14, e.g. in one or more of the regions 72-76, 131-175, 133-145, 158-165, 228-238, 231-237 or 255-262 of VP2.

In a further embodiment, the peptide has been inserted in VP3 such as in VP3 of HRV14, e.g. in one or more of the regions 54-95, 57-64, 72-77, 193-212, 196-204 or 226-236 of VP3.

In a further embodiment, the peptide has been inserted in VP4 such as in VP4 of HRV14.

In addition to, or as an alternative for, the insertion of a peptide, the VLP may comprise at least one modification which results in attenuation of an immunodominant epitope. Immunodominant epitopes are often hypervariable and thus by attenuating such epitopes, the immune response may become more directed to other, more conserved, epitopes on the capsid.

In one such embodiment, VP0 comprises said modification, e.g. wherein VP0 has been modified so that the NIm-II site epitope has been attenuated and/or wherein one or more residues in one or more of the regions corresponding to 131-175, 228-238 or 255-262 in VP2 has been modified, e.g. wherein a residue corresponding to residue 158, 159, 161 and/or 162 has been modified.

In another such embodiment, VP1 comprises said modification, e.g. wherein VP1 has been modified so that the NIm-IA site epitope has been attenuated and/or VP1 has been modified so that the NIm-IB site epitope has been attenuated and/or wherein one or more residues in one or more of the regions 73-102, 129-149, 204-212 and 264-289 has been modified, for example wherein residue 91 and/or 95 has been modified or wherein residue 83, 85, 138 and/or 139 has been modified.

In another such embodiment, VP2 comprises said modification, e.g. wherein VP2 has been modified so that the NIm-II site epitope has been attenuated and/or wherein one or more residues in one or more of the regions 131-175, 228-238 or 255-262 has been modified, e.g wherein residue 158, 159, 161 and/or 162 has been modified.

In another such embodiment, VP3 comprises said modification, e.g. wherein VP3 has been modified so that the NIm-III site epitope has been attenuated and/or wherein one or more residues in one or more of the regions 54-95, 193-212 or 226-236 has been modified, e.g. wherein residue 72, 75 and/or 78 has been modified.

In another such embodiment, VP4 comprises said modification.

Regardless of whether the above-described modifications involve insertions, deletions or substitutions of amino acids, the capsid protein sequences will remain similar to the naturally-occurring protein sequences from which they are derived. The VLP may contain a mixture of unmodified and modified capsid proteins.

In one embodiment, the VP0 protein is the HRV14 VP0 protein or a variant thereof, wherein the variant has more than 90% sequence identity to HRV14 VP0.

In another embodiment, the VP0 protein is the HRV14 VP0 protein or a variant thereofhaving 1-25, such as 1-10 amino acid additions or deletions at either end and/or 1-50, such as 1-25, e.g. 1-15 amino acid substitutions or additions or deletions within the sequence.

In another embodiment, the VP1 protein is the HRV14 VP1 protein or a variant thereof, wherein the variant has more than 90% sequence identity to HRV14 VP1.

In another embodiment, the VP1 protein is the HRV14 VP1 protein or a variant thereofhaving 1-25, such as 1-10 amino acid additions or deletions at either end and/or 1-50, such as 1-25, e.g. 1-15 amino acid substitutions or additions or deletions within the sequence.

In another embodiment, the VP2 protein is the HRV14 VP2 protein or a variant thereof, wherein the variant has more than 90% sequence identity to HRV14 VP2.

In another embodiment, the VP2 protein is the HRV14 VP2 protein or a variant thereofhaving 1-25, such as 1-10 amino acid additions or deletions at either end and/or 1-50, such as 1-25, e.g. 1-15 amino acid substitutions or additions or deletions within the sequence.

In another embodiment, the VP3 protein is the HRV14 VP3 protein or a variant thereof, wherein the variant has more than 90% sequence identity to HRV14 VP3.

In another embodiment, the VP3 protein is the HRV14 VP3 protein or a variant thereofhaving 1-25, such as 1-10 amino acid additions or deletions at either end and/or 1-50, such as 1-25, e.g. 1-15 amino acid substitutions or additions or deletions within the sequence.

In another embodiment, the VP4 protein is the HRV14 VP4 protein or a variant thereof, wherein the variant has more than 90% sequence identity to HRV14 VP4.

In another embodiment, the VP4 protein is the HRV14 VP4 protein or a variant thereofhaving 1-25, such as 1-10 amino acid additions or deletions at either end and/or 1-50, such as 1-25, e.g. 1-15 amino acid substitutions or additions or deletions within the sequence.

As described above, in a further aspect, the invention relates to an immunogenic composition comprising a VLP of the invention and a pharmaceutically acceptable diluent, excipient or carrier. Pharmaceutically acceptable carriers and excipients are well known and can be selected by those of skill in the art. For example, the carrier or excipient can favorably include a buffer. Optionally, the carrier or excipient also contains at least one component that stabilizes solubility and/or stability. Examples of solubilizing/stabilizing agents include detergents, for example, laurel sarcosine and/or tween. Alternative solubilizing/stabilizing agents include arginine, and glass forming polyols (such as sucrose, trehalose and the like). Numerous pharmaceutically acceptable carriers and/or pharmaceutically acceptable excipients are known in the art and are described, *e.g.,* in Remington's Pharmaceutical Sciences, by E. W. Martin, Mack Publishing Co., Easton, PA, 5th Edition (975).

Accordingly, suitable excipients and carriers can be selected by those of skill in the art to produce a formulation suitable for delivery to a subject by a selected route of administration.
Suitable excipients include, without limitation: glycerol, Polyethylene glycol (PEG), Sorbitol, Trehalose, N-lauroylsarcosine sodium salt, L -proline, Non detergent sulfobetaine, Guanidine hydrochloride, Urea, Trimethylamine oxide, KCl, Ca²⁺, Mg²⁺ , Mn²⁺ , Zn²⁺ and other divalent cation related salts, Dithiothreitol, Dithioerytrol, and β-mercaptoethanol. Other excipients can be detergents (including: Tween80, Tween20, Triton X-00, NP-40, Empigen BB, Octylglucoside, Lauroyl maltoside, Zwittergent 3-08, Zwittergent 3-0, Zwittergent 3-2, Zwittergent 3-4, Zwittergent 3-6, CHAPS, Sodium deoxycholate, Sodium dodecyl sulphate, Cetyltrimethylammonium bromide).

In one embodiment, the composition comprises 2, 3, 4 or more different VLPs of the invention as described herein.

One important aspect of a vaccine against rhinovirus is that it will protect against a sufficient number of rhinovirus serotypes to provide effective protection against rhinovirus infection. Thus, a combination of the above-described embodiments may provide an optimal composition. For example, the immunogenic composition of the invention may contain multiple VLPs originating from different serotypes, and/or VLPs comprising capsin proteins originating from different serotypes, and/or modified capsin proteins wherein one or more conserved peptides have been inserted, and/or modified capsin proteins wherein one or more immunodominant epitopes have been attenuated.

In another embodiment, the immunogenic composition further comprises an adjuvant, such as a Thl adjuvant. Suitable adjuvants include suspensions of minerals (alum, aluminium hydroxide, aluminium phosphate) onto which antigen is adsorbed; emulsions, including water-in-oil, and oil-in-water (and variants thereof, including double emulsions and reversible emulsions), liposaccharides, lipopolysaccharides, immunostimulatory nucleic acids (such as CpG oligonucleotides), liposomes, Toll-like Receptor agonists (particularly, TLR2, TLR4, TLR7/8 and TLR9 agonists), and various combinations of such components.

In a preferred embodiment, the adjuvant is an aluminium salt, such as aluminium hydroxide. In another preferred embodiment, the adjuvant comprises a TLR4 agonist, such as 3-O-deacylated monophosphoryl lipid A (3D-MPL) and/or a saponin, such as QS21 (WO 96/33739), preferably in a liposomal formulation.

In another embodiment, the immunogenic composition comprises further antigens derived from pathogens other than HRV, such as Moraxella catarrhalis (MCat) or nontypeable Haemophilus influenzae (NTHI). Further inclusion of antigens directed at MCat and/or NTHI are particularly contemplated in the prevention or treatment of COPD.

In one aspect, the invention relates to the VLP or immunogenic composition as described herein for use in the prevention or treatment in humans of a rhinoviral infection, such as prevention or treatment of common cold.

In another aspect, the invention relates to the VLP or immunogenic composition as described herein for use in induction of an immune response involving neutralising antibodies and/or a cellular response, against rhinovirus in humans. In one embodiment the neutralising antibodies are cross-neutralising antibodies.

The present disclosure also relates to the use of a VLP or an immunogenic composition as described herein, in the manufacture of a medicament for the prevention or treatment in humans of rhinoviral infection, such as prevention or treatment of common cold.

The present disclosure also relates to the use of a VLP or an immunogenic composition as described herein, in the manufacture of a medicament inducing an immune response involving neutralising antibodies and/or a cellular response, against rhinovirus in humans. In one embodiment the neutralising antibodies are cross-neutralising antibodies.

The present disclosure also relates to a method for inducing an immune response, such as an immune response involving neutralising antibodies and/or a cellular response, against rhinovirus in humans comprising administering to a human a VLP or an immunogenic composition as described herein.

The present disclosure also relates to a method for inducing an immune response, such as an immune response involving cross-neutralising antibodies and/or a cellular response, against rhinovirus in humans comprising administering to a human a VLP or an immunogenic composition as described herein.

The present disclosure also relates to a method for preventing rhinovirus infection or disease related to rhinovirus, such as common cold, COPD or asthma exacerbations, which method comprises administering to a human a VLP or an immunogenic composition as described herein.

In another aspect, the invention relates to the VLP or the immunogenic composition as described herein for use in the prevention or treatment of COPD or asthma exacerbations in humans.

The present disclosure also relates to the use of a VLP or an immunogenic composition as described herein, in the manufacture of a medicament for the prevention or treatment of COPD or asthma exacerbations in humans.

The immunogenic compositions may be for eliciting an immune response against HRV in human infants (e.g., infants between birth and 1 year, such as between 0 and 6 months, at the age of initial dose). Or in another embodiment, immunogenic compositions may be for eliciting an immune response against HRV in elderly humans. Or the immunogenic composition may be for administration to adults or children. It will be appreciated that the choice of adjuvant can be different in these different applications, and the optimal adjuvant and concentration for each situation can be determined empirically by those of skill in the art.

The immunogenic compositions described herein can be administered as vaccines by any of a variety of routes. Intramuscular, sublingual and intradermal deliveries are preferred.

The dosage of the VLPs can vary with the condition, sex, age and weight of the individual and the administration route of the vaccine. The quantity can also be varied with the number of different VLPs.

Typically, the amount of protein in each dose of the immunogenic composition is selected as an amount which induces an immunoprotective response without significant, adverse side effects in the typical subject. Immunoprotective in this context does not necessarily mean completely protective against infection; it means protection against symptoms or disease, especially severe disease associated with the virus. The amount of antigen can vary depending upon which specific immunogen is employed. Generally, it is expected that each human dose will comprise 1 -1000µg of protein. Suitably each vaccine dose comprises 1-100 µg of each VLP, suitably at least 5µg, or at least 10 µg, for example, between 5- 50 µg of each VLP.

The immunogenic compositions described herein suitably generate an immune response in a human or animal subject against at least 2 different rhinoviruses or two different serotypes of a rhinovirus such as two different HRV serotypes, suitably 2 or more, 3 or more, 4 or more, 5 or more, or 10 or more different HRV serotypes. Cross-protection against different HRV serotypes can e.g. be identified using an animal model, for example mouse models (Bartlett *et al* 2008).

Suitably the immunogenic composition is delivered in a 2 or 3 dose regimen, for example in a 0, 1 or a 0, 2 or a 0, 3 or a 0, 4 or a 0, 5 or a 0, 6 or a 0, 12 month regimen, or 0, 1, 6 or a 0, 2, 6 or a 0, 6, 12 month regimen respectively.

### Methods for producing rhinoviral VLPs

In one embodiment, the VLP of the invention is produced by a method for producing rhinoviral VLPscomprising the steps of:
a. producing constructs encoding capsid proteins required for the formation of a VLP, wherein each of the capsid proteins is encoded as a fusion protein with a chaperone protein,
b. co-expressing said constructs in the same host cell or expressing one or more of said constructs in separate host cells, and
c. lysing said host cells and, in case of separate expression, bringing together all capsid proteins required for a VLP.

The term "chaperone protein" or "chaperone sequence" when used herein refers to a protein that assists other proteins to fold properly and stabilizes proteins. Typically, proper folding and stabilisation leads to improved solubility. Examples of such chaperone proteins are SUMO and Hsp90. In one embodiment, said chaperone protein is SUMO or Hsp90, preferably SUMO. In an alternative embodiment, constructs using SUMO as chaperone and constructs using Hsp90 as chaperone are both co-expressed.

In one embodiment, the method further comprises step d. removing said chaperone protein. In a specific embodiment, the chaperone protein is SUMO. Removal of SUMO in step d. can be, but is not necessarily, done using SUMOstar protease.

Methods for production of nucleic acid constructs are well known in the art. In certain embodiments, the recombinant nucleic acids that encode the proteins are codon optimized for expression in a selected host cell. To facilitate replication and expression, the nucleic acid constructs that encode the proteins can be incorporated into a vector, such as a prokaryotic or a eukaryotic expression vector.

Suitable host cells include prokaryotic (i.e., bacterial) host cells, such as *E. coli,* as well as numerous eukaryotic host cells, including fungal (e.g., yeast) cells, insect cells, plant cells, and mammalian cells (such as CHO and HEK293 cells). Preferred host cells are eukaryotic cells, e.g. mammalian cells, in particular HEK293 cells.

The host cells can be cultured in conventional nutrient media modified as appropriate for activating promoters, selecting transformants, or amplifying the inserted polynucleotide sequences. The culture conditions, such as temperature, pH and the like, are typically those previously used with the host cell selected for expression, and will be apparent to those skilled in the art and in the references cited herein, including, e.g., Freshney (1994) Culture of Animal Cells, a Manual of Basic Technique, third edition, Wiley- Liss, New York and the references cited therein. In addition to Sambrook, Berger and Ausubel, details regarding cell culture can be found in Payne et al. (1992) Plant Cell and Tissue Culture in Liquid Systems John Wiley & Sons, Inc. New York, NY; Gamborg and Phillips (eds) (1995) Plant Cell, Tissue and Organ Culture; Fundamental Methods Springer Lab Manual, Springer-Verlag (Berlin Heidelberg New York) and Atlas and Parks (eds) The Handbook of Microbiological Media (1993) CRC Press, Boca Raton, FL.

Typically, the method for producing VLPs as described herein above will further comprise one or more purification steps, e.g. a purification step between steps c. and d. and/or a purification step after step d.

Addition of a tag to the fusion protein can facilitate purification. In one embodiment, the fusion protein comprises a histidine tag. Proteins comprising a histidine tag can be purified using immobilized metal affinity chromatography. Thus, in one embodiment, the method comprises a purification step which comprises immobilized metal affinity chromatography.

The term "Hsp90" when used herein refers to heat shock protein 90. Alternatives include other members of the 90-kDa molecular chaperone family, such as described by Csermely P. et al. ("The 90-kDa Molecular Chaperone Family: Structure, Function, and Clinical Applications. A Comprehensive Review" Pharmacol. Ther. Vol. 79, No. 2, pp. 129-168, 1998).

A "SUMO sequence" or "SUMO" when used herein refers to a Small Ubiquitin-related Modifier sequence or protein. SUMO sequences, like other chaperone proteins, enhance the solubility of expressed fusion proteins. Suitable SUMO sequences have been described in Ulrich "SUMO Protocols" Methods in Mol Biol 497. Humana Press 2009 and include SUMO-1, SUMO-2, SUMO-3, SUMO-4, Smt3 and Pmt3. A pET SUMO Expression System for bacterial expression is e.g. available from Life Technologies. SUMO fusion technology has been review by Panavas et al. 2009 Methods Mol Biol 497:303.

In a preferred embodiment, the SUMO sequence is Smt3.
In one embodiment, the SUMO sequence is removed using SUMOprotease.

Suitably, one of the constructs encodes VP0 and said construct encodes the sequence set forth in SEQ ID NO:2.

Suitably, the construct encoding VP3 encodes the sequence set forth in SEQ ID NO:4.

Suitably, the construct encoding VP1 encodes the sequence set forth in SEQ ID NO:6.

### EXAMPLES

### MATERIALS AND METHODS

### 1. Generation of the expression plasmids

### Codon optimization

The Rhinovirus genomic sequence HRV14 from NCBI (accession number NC_001490) was used as a reference. The portion of the HRV14 genomic sequence coding for the P1 structural protein precursor was codon optimized to align with Homo sapiens codon usage table in order to facilitate expression in the human derived selected expression host which is the Human Embryonic Kidney 293 (HEK293) cell line. Although nucleotidic sequence was optimized, none of these substitutions resulted in amino acid sequence alteration.

### Construct assembly

The same procedure applies to the three chimeric SUMO-VPs constructs (SUMO-VP0 (SEQ ID NO:1, encoding SEQ ID NO:2), SUMO-VP3 (SEQ ID NO:3, encoding SEQ ID NO:4) and SUMO-VP1 (SEQ ID NO:5, encoding SEQ ID NO:6)) generated for the experiment.

The DNA fragment coding for the chimeric SUMO-VP was generated by the assembly of two smaller DNA fragments. The first DNA fragment consisted of the entire coding sequence for the SUMOstar fusion protein and a portion of the structural HRV VP protein. The second fragment was PCR amplified from a codon optimized HRV14 DNA template. The two fragments were assembled using PCR.

### Molecular cloning

The assembled fragment and eukaryotic expression vector pTT5 (Zhang et al. (2009) Protein Expr Purif 65:77) were used to generate the expression plasmids. Procedures sufficient to guide one of skill in the art can be found in the following references: Sambrook et al., Molecular Cloning: A Laboratory Manual, Cold Spring Harbor Laboratory Press, Cold Spring Harbor, NY, 2000; and Ausubel et al. Short Protocols in Molecular Biology, 4th ed., John Wiley & Sons, Inc., 1999.

### 2. Description of protein expression conditions

### Cell maintenance

The experiment was conducted in a mammalian expression system. The cells were grown in suspension in F17 animal origin-free, chemically defined, protein-free expression medium (Life technologies, CA, USA) supplemented with 4mM Glutamine and 0.1% Pluronic F-68 at 37°C, 5% CO₂ and 120rpm. Passages were done by dilution in fresh medium regularly. Cells were maintained within the 0.2 - 4.0X10⁶ cells/mL range of viable cell density. Cell density and viability were monitored using the Cellometer cell counter (Nexcelom Bioscience).

### Transient transfection

Following is an example of a transient transfection at a 30mL scale. Volumes were scaled up accordingly for larger production up to 4 liters. The same culture medium (F17 animal origin-free, chemically defined, protein-free expression medium supplemented with 4mM Glutamine and 0.1% Pluronic F-68) as in the hereabove cell maintenance section was used for the transfection step.

Briefly, the day before the transfection, a 125mL cell culture shake flask was seeded with 30mL of HEK293-6E cells at a 5.0X10⁵ cells/mL viable cell density and were allowed to grow overnight at 37°C, 5% CO₂ 110-120 rpm. On the transfection day, cell density was adjusted at 1.0X10⁶ cells/mL. Transfection mixes were prepared as followed; 12.5 µg of transfection grade expression plasmids His-SUMO-VPO, His-SUMO-VP3 and His-SUMO-VP1 (total of 37.5ug of expression plasmids) were diluted in Opti-Pro™ SFM buffer (Life technologies, CA, USA) to a total volume of 0.6 mL. Similarly, 37.5 µL of FreeStyle Max transfection reagent was also diluted in Opti-Pro™ SFM to a total volume of 0.6 mL. Diluted DNA and transfection reagent were then mixed together and incubated at room temperature for 10 minutes. After the incubation time, the transfection mix was added slowly to the cells and put back in culture at 37°C, 5% CO₂ 110-120 rpm for a 6-day expression period. At that time, cells were harvested by centrifugation at 6000 x g for 10 mins at 4°C.

### 3. Description of protein purification

### Cell lysis

Cell pellet corresponding to 2L culture, was resuspended in 50ml of 20 mM Bicine buffer (pH 8.3) containing 0.5M NaCl. Cell suspension was disrupted, using a TS Bench Top Constant Cell Disrupter System 1.1 KW (Constant Systems, Ltd), by two passages at 15 000 PSI (pounds per square inch). Soluble (supernatant) and insoluble (pellet) material were separated by centrifugation at 20 000 x g for 20 min at 4°C.

### IMAC purification step

Soluble material containing the three co-expressed chimeric SUMO-VPs was purified under native conditions on immobilized metal affinity chromatography (IMAC) using protein purification system (PROFINIA™ Bio-Rad Laboratories, Inc. or AKTA system, GE Healthcare, Inc.). The protein preparation was loaded at 2ml/min on 5ml His Trap FF columns (GE Healthcare, Inc.) pre-equilibrated with 20 mM Bicine buffer (pH 8.3), 0.5M NaCl, (producing a "flow through fraction"). Thereafter, the column was washed at 10 ml/min flow rate, using 5 column volumes (CV) of the same buffer (producing a "wash fraction #1"), followed by 5CV of the buffer supplemented with 10mM imidazole (producing a "wash fraction #"2). Elution step was performed using two CV of 20 mM Bicine buffer (pH 8.3), 0.5M NaCl, 250 mM imidazole and at a flow rate of 2 ml/min, producing an "elution fraction".

### SEC purification step

A fraction of the IMAC "elution fraction" was loaded on a size exclusion chromatography (SEC) column (HiLoad™ Superdex™ 200pg, GE Healthcare, Inc) preequilibrated in 20 mM Bicine buffer (pH 8.3) containing 0.5M NaCl, with and without 1 mM TCEP, at 2.5ml/min. Fractions were analyzed and pooled according to sodium dodecyl sulfate polyacrylamide gel electrophoresis (SDS-PAGE) results. If required, proteins were concentrated using Amicon Ultra Centrifugal Filter Unit- 10,000 NMWL (EMD Millipore, Inc.).

### 4. Use of the SUMOstar protease

Purified chimeric SUMO-VPs proteins were treated with the SUMOstar protease (SUMOstar kit (cat#7110) from Life Sensors) in order to remove the His-SUMO protein fusion parts and liberate the VP proteins. The SUMOstar protease was added to the protein preparation according to the recommended ratio (1U/100µg) in presence of SUMOstar protease buffer and 1 to 5 mM DTT and incubated at 30°C, for more than 60 minutes. Proteolysis experiments could be performed in presence of IMAC resin (IMAC sepharose 6 FF, GE Healthcare, Inc).

### 5. Description of the characterisation of the particles

### Sample preparation for SDS-PAGE

For example, 1 ml of culture was centrifuged at 14 000 RPM for 2 min. The cell pellet was resolubilized using 140 or 210 µl of 8M urea buffer, 10 or 15 µl of dithiothreitol (DTT) 1M and 50 or 75 µl of NUPAGE® LDS (Lithium Dodecyl Sulphate) Sample Buffer 4X (Life Technologies). The cells in suspension were lysed by sonication, with 10 to 15 cycles of 2 seconds of sonication at an output watt of 0.08, amplitude of 40-50, followed by a pause step of one second (Vibra Cell, Sonics & Materials, Inc.). The cell lysates were then centrifuged at 14 000 RPM for 2 min to separate the insoluble components. Thereafter, samples were heated at 70°C for 10 minutes.

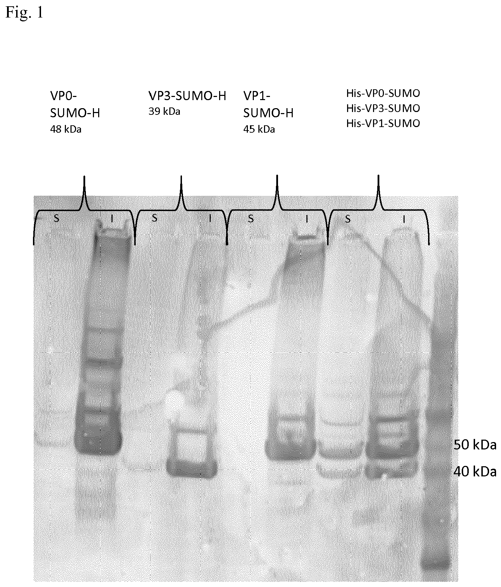

Alternatively, when desired to see the effect of SUMO sequence on the solubility of the co-expressed proteins, for example as in the case for Fig. 1, samples were prepared as follows. Cells contained in 1ml of culture were isolated by centrifugation at 5000g for 5 mins. The pelleted cells were resuspended in 200µl of 20 mM of Bicine and 150 ml NaCl, and, sonicated. Soluble and insoluble protein were separated by centrifugation at 14000 rpm for 10 mins. The pellet obtained was resuspended in 200 µl of 20 mM Bicine, 8M urea, and, sonicated. The soluble part was thus contained in the supernatant.

Purified proteins were prepared for SDS-PAGE analysis by adding 70 ul of sample, 5 ul of DTT 1M and 25 ul of LDS Sample Buffer 4X.

### SDS-PAGE analysis

SDS-PAGE analyses were performed according to manufacturer's recommendations (Life Technologies) using NUPAGE® Bis-Tris 4-12% gels. Preparations of samples, buffers and migration conditions were done under conditions recommended by the suppliers.

### DLS

The oligomerization state of VLP-VP-SUMO was evaluated by dynamic light scattering (DLS) which is used to evaluate hydrodynamic radius in solution, to provide information about homogeneity and to detect presence of high molecular weight particles. This is based on the calculation of the diffusion coefficient of the different species that are obtained by measuring the light scattering fluctuation, which depends on protein molecular size and shape, and on the other minor constituents of the sample. Briefly, samples obtained from the SEC purification step were centrifuged at 20 000g for 2 minutes before being charged in Low volume 384 well black clear flat bottom Corning 3540 plate and subject to DLS analysis using a DynaPro® Plate reader from Wyatt Technology at 25°C. For each sample analyzed by DLS, five consecutive wells were loaded with the same sample and six consecutive measurements were taken on the same well and measured with a light-scattering data collection of 15s. Analyzing particle size distribution by cumulant or regularization fit and polydispersity index were calculated from the correlation function using Dynamics Software version 7.1.7 (Wyatt Technology).

### TEM

Samples obtained from the SEC purification step were prepared for TEM (transmission electron microscopy) negative staining using phosphotungstic acid 3% as contrasting agent and samples were adsorbed on TEM grids (200 mesh) using an Airfuge (an air-driven ultracentrifuge made by Beckman). The material was left to dry completely and examined by transmission electron microscopy (Hitachi H-7100) at 75kV.

### RESULTS

### Expression in HEK293

Three expression plasmids (His-SUMO-VPO, His-SUMO-VP3 and His-SUMO-VP1) were used to simultaneously and individually transfect HEK293-6E cells. After the 6 day expression period, the transfected HEK293-6E cells were harvested, resuspended and lysed. Soluble and insoluble fractions were loaded on LDS-PAGE and Western transferred. Western blot analysis using rabbit anti-His polyvalent antibody revealed that individually all 3 His-SUMO-VPs were expressed at a relatively high level but mainly in the insoluble fraction. On the other hand, solubility improved when the 3 His-SUMO-VPs were coexpressed (**Figure 1**).

### Purification step 1

Total HEK293 pellet was resuspended in a suitable buffer to keep the proteins in a native condition (no denaturation). The cell suspension was disrupted and phases were separated by centrifugation. The soluble fraction was used to perform a conventional IMAC purification under native conditions.

### Purification step 2

The IMAC purified proteins / particles under native conditions were submitted to an additional purification step. By performing a size exclusion chromatography (SEC) step, we aimed at separating the loaded material based on its size in solution. Samples from different fractions containing His-SUMO-VPs were pooled and characterized for their content of particles.

### Dynamic Light Scattering (DLS) and Transmission Electronic Microscopy (TEM)

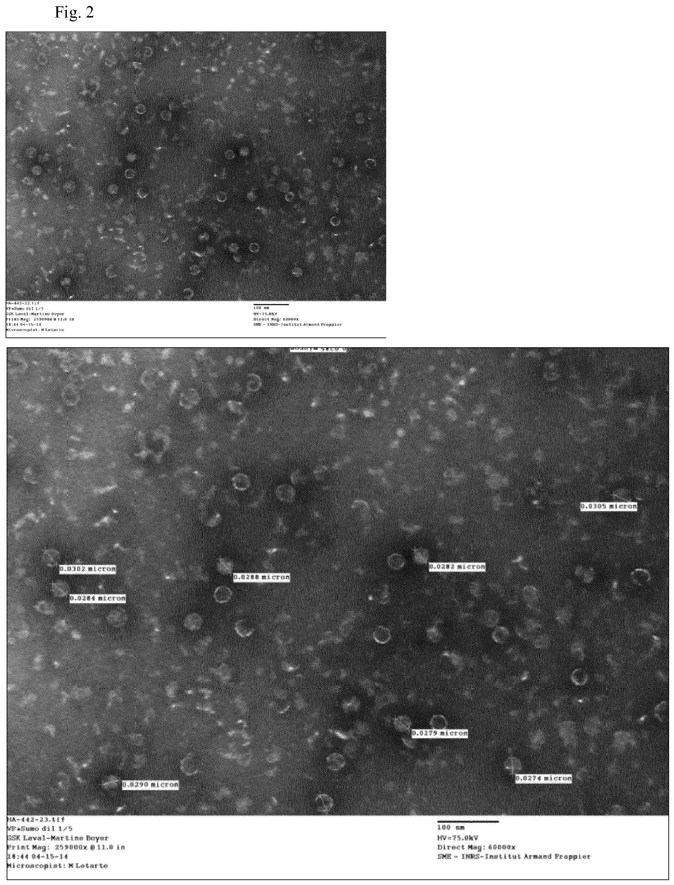

The different pooled fractions were analysed in parallel in DLS (**Table 1**) and TEM (**Figure 2**).

**Table 1. Diameter results from batch mode Dynamic light scattering experiment.**

| | Cumulant fit | Regularization fit | | | |
|---|---|---|---|---|---|
| Sample | Diameter (nm) | Diameter (nm) | % Mass | % Polydispersity | % Intensity |
| His-SUMO-VPs | 37.0 ± 8.6 | 27.6 ± 1.4 | 94.8 | 34.6 | 97.8 |

The measured hydrodynamic diameter of 27.6nm is coherent with the expected size range of a virus like particle (VLP). Also, the high mass and intensity percentage of the target population and the low hydrodynamic diameter of the overall sample (cumulant fit) suggest a good sample homogeneity.

Both methods gave us good indications that structures were obtained in the range of 30nm in diameter (**Table 2**).

**Table 2. Summary of diameters and widths of distribution and comparison between TEM and DLS**

| | DLS hydrodynamic diameter | TEM external diameter |
|---|---|---|
| Sample | Mean (nm) | Mean (nm) |
| His-SUMO-VPs | 27.6 ± 1.4 | 30 ± 3 |

### SUMOprotease processing

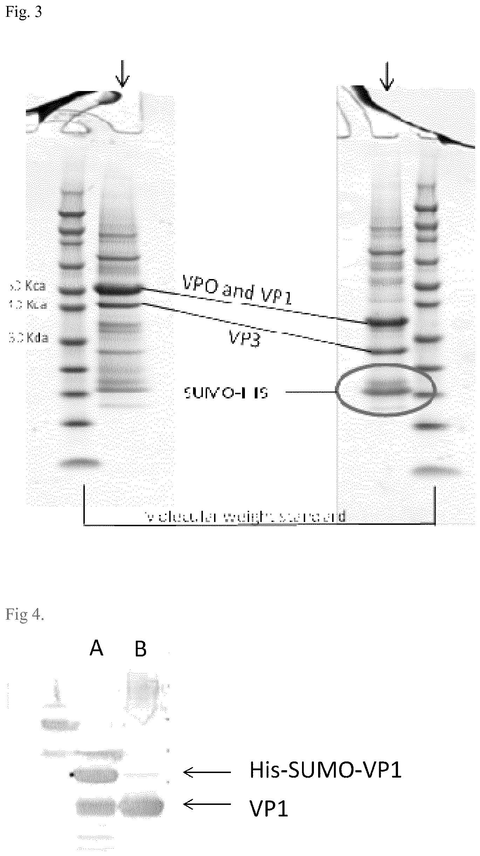

The data described above were generated using the His-SUMO-VPs purified proteins. In vitro digestion of the His-SUMO-VPs proteins using SUMOstar protease was performed to liberate the His-SUMO from the VPs portion. As a result, a clear indication that a good proportion of the His-SUMO-VPs were properly cleaved (into His-SUMO and VPs) was apparent from the fact that the new bands observed in the right portion of Figure 3 correspond in molecular weight to the processed proteins. Western blot analysis using anti-His and anti-VPl were performed and confirmed that result (Figure 4). Figures 3 and 4 also appear to show some cleavage of the His-SUMO from the VPs portion prior to the digestion using SUMO protease.

### Sequence listing

SEQ ID NO:1
   Nucleotide sequence encoding HRV14 SUMO-VPO (LVL1053-43)
SEQ ID NO:2
   Amino acid sequence of HRV14 SUMO-VPO (LVL1053-43)
SEQ ID NO:3
   Nucleotide sequence encoding HRV14 SUMO-VP3 (LVL1054-21)
SEQ ID NO:4
   Amino acid sequence of HRV14 SUMO-VP3 (LVL1054-21)
Seq ID NO:5
   Nucleotide sequence encoding HRV14 SUMO-VP1 (LVL1055-12)
SEQ ID NO:6
   Amino acid sequence of HRV14 SUMO-VP1 (LVL1055-12)
SEQ ID NO: 14
   >sp|P03303|2-331 - VP0 - HRV14
SEQ ID NO: 15
   >sp|P03303|568-856 - VP1 - HRV14
SEQ ID NO: 16
   >sp|P03303|70-331 - VP2 - HRV14
SEQ ID NO: 17
   >sp|P03303|332-563 - VP3 - HRV14
SEQ ID NO: 18
   >sp|P03303|2-69 - VP4 - HRV14

## Claims

1. A Virus-Like Particle (VLP), comprising human rhinovirus capsid proteins VP0, VP1 and VP3, or, comprising human rhinovirus capsid proteins VP1, VP2, VP3 and VP4.

2. The VLP of claim 1, wherein all capsid proteins originate from the same seroptype of human rhinovirus.

3. The VLP of claim 1, wherein the capsid proteins originate from two or more different serotypes of human rhinovirus.

4. The VLP of any preceding claim, wherein one or more of the capsid proteins comprises at least one modification which increases the ability of the VLP to induce a cross-reactive immune response against multiple human rhinoviral serotypes, as compared to a VLP wherein said modification is not present, where said modification consists of insertion of at least one peptide from a rhino viral capsid protein, said peptide capable of inducing a cross-reactive immune response against two or more human rhinoviral serotypes.

5. The VLP of any one of the preceding claims, wherein the VLP comprises at least one modification which results in attenuation of an immunodominant epitope.

6. The VLP of any one of the preceding claims, wherein the VPO protein has a sequence selected from the group consisting of(a) SEQ ID NO:14; (b) a sequence having more than 90% sequence identity to SEQ ID NO: 14, (c) SEQ ID NO: 14 having from 1-25 amino acid additions or deletions at either end, and (d) SEQ ID NO:14 having 1-50 amino acid substitutions or deletions within the sequence.

7. The VLP of any one of the preceding claims, wherein the VP1 protein has a sequence selected from the group consisting of(a) SEQ ID NO: 15, (b) a sequence having more than 90%sequence identity to SEQ ID NO: 15, (c) SEQ ID N0:15 having from 1-25 amino acid additions or deletions at either end, and (d) SEQ ID NO: 15 having 1-50 amino acid substitutions or deletions within the sequence.

8. The VLP of any one of the preceding claims, wherein the VP2 protein has a sequence selected from the group consisting of(a) SEQ ID NO: 16, (b) a sequence having more than 90% sequence identity to SEQ ID NO: 16, (c) SEQ ID NO: 16 having from 1-25 amino acid additions or deletions at either end, and (d) SEQ ID NO: 16 having 1-50 amino acid substitutions or deletions within the sequence.

9. The VLP of any one of the preceding claims, wherein the VP3 protein has a sequence selected from the group consisting of(a) SEQ ID NO: 17, (b) a sequence having more than 90% sequence identity to SEQ ID NO: 17, (c) SEQ ID NO: 17 having from 1-25 amino acid additions or deletions at either end, and (d) SEQ ID NO: 17 having 1-50 amino acid substitutions or deletions within the sequence.

10. The VLP of any one of the preceding claims, wherein the VP4 protein has a sequence selected from the group consisting of (a) SEQ ID NO: 18, (b) a sequence having more than 90% sequence identity to HRV14 VP4 SEQ ID NO: 18, (c) SEQ ID NO: 18 having from 1-25 amino acid additions or deletions at either end, and (d) SEQ ID NO: 18 having 1-50 amino acid substitutions or deletions within the sequence.

11. An immunogenic composition comprising the VLP of any one of the preceding claims and a pharmaceutically acceptable diluent, excipient or carrier.

12. The VLP of any one of the claims 1 to 10 or the immunogenic composition of claim 11 for use in a method for inducing an immune response in a human against human rhinovirus.

13. The VLP of any one of claims 1 to 10 or the immunogenic composition of claim 11 wherein the VLP is produced by a method for producing rhinoviral VLPs comprising the steps of:
a. producing constructs encoding capsid proteins required for the formation of a VLP, wherein each of the capsid proteins is encoded as a fusion protein with a chaperone protein,
b. co-expressing said constructs in the same host cell or expressing one or more of said constructs in separate host cells, and,
c. lysing said host cells and bringing, in case of separate expression, all capsid proteins required for a VLP together.

14. The VLP or the immunogenic composition of claim 13, wherein the method further comprises step d. removing said chaperone protein.

15. The VLP or the immunogenic composition of claim 13 wherein the chaperone protein is a Small Ubiquitin-related Modifier (SUMO) protein.

## Patentansprüche

1. Virus-ähnliches Partikel (Virus-Like Particle, VLP), das die Kapsidproteine VP0, VP1 und VP3 von humanem Rhinovirus umfasst, oder das die Kapsidproteine VP1, VP2, VP3 und VP4 von humanem Rhinovirus umfasst.

2. VLP gemäß Anspruch 1, wobei alle Kapsidproteine von dem gleichen Serotyp von humanem Rhinovirus stammen.

3. VLP gemäß Anspruch 1, wobei die Kapsidproteine von zwei oder mehr verschiedenen Serotypen von humanem Rhinovirus stammen.

4. VLP gemäß irgendeinem vorherigen Anspruch, wobei eins oder mehrere der Kapsidproteine mindestens eine Modifikation umfasst/umfassen, die die Fähigkeit des VLPs erhöht, eine kreuzreaktive Immunantwort gegen mehrere human-rhinovirale Serotypen zu induzieren, im Vergleich zu einem VLP, in dem die Modifikation nicht vorhanden ist, wobei die Modifikation aus der Insertion mindestens eines Peptids von einem rhinoviralen Kapsidprotein besteht, wobei das Peptid fähig ist, eine kreuzreaktive Immunantwort gegen zwei oder mehr human-rhinovirale Serotypen zu induzieren.

5. VLP gemäß irgendeinem der vorherigen Ansprüche, wobei das VLP mindestens eine Modifikation umfasst, die zu einer Attenuierung eines immundominanten Epitops führt.

6. VLP gemäß irgendeinem der vorherigen Ansprüche, wobei das VP0-Protein eine Sequenz aufweist, die ausgewählt ist aus der Gruppe, bestehend aus (a) SEQ ID NO: 14; (b) einer Sequenz mit mehr als 90 % Sequenzidentität zu SEQ ID NO: 14, (c) SEQ ID NO: 14 mit von 1-25 Aminosäureadditionen oder -deletionen an einem der Enden und (d) SEQ ID NO: 14 mit 1-50 Aminosäuresubstitutionen oder -deletionen innerhalb der Sequenz.

7. VLP gemäß irgendeinem der vorherigen Ansprüche, wobei das VP1 -Protein eine Sequenz aufweist, die ausgewählt ist aus der Gruppe, bestehend aus (a) SEQ ID NO: 15, (b) einer Sequenz mit mehr als 90 % Sequenzidentität zu SEQ ID NO: 15, (c) SEQ ID NO: 15 mit von 1-25 Aminosäureadditionen oder -deletionen an einem der Enden und (d) SEQ ID NO: 15 mit 1-50 Aminosäuresubstitutionen oder -deletionen innerhalb der Sequenz.

8. VLP gemäß irgendeinem der vorherigen Ansprüche, wobei das VP2-Protein eine Sequenz aufweist, die ausgewählt ist aus der Gruppe, bestehend aus (a) SEQ ID NO: 16, (b) einer Sequenz mit mehr als 90 % Sequenzidentität zu SEQ ID NO: 16, (c) SEQ ID NO: 16 mit von 1-25 Aminosäureadditionen oder -deletionen an einem der Enden und (d) SEQ ID NO: 16 mit 1-50 Aminosäuresubstitutionen oder -deletionen innerhalb der Sequenz.

9. VLP gemäß irgendeinem der vorherigen Ansprüche, wobei das VP3-Protein eine Sequenz aufweist, die ausgewählt ist aus der Gruppe, bestehend aus (a) SEQ ID NO: 17, (b) einer Sequenz mit mehr als 90 % Sequenzidentität zu SEQ ID NO: 17, (c) SEQ ID NO: 17 mit von 1-25 Aminosäureadditionen oder -deletionen an einem der Enden und (d) SEQ ID NO: 17 mit 1-50 Aminosäuresubstitutionen oder -deletionen innerhalb der Sequenz.

10. VLP gemäß irgendeinem der vorherigen Ansprüche, wobei das VP4-Protein eine Sequenz aufweist, die ausgewählt ist aus der Gruppe, bestehend aus (a) SEQ ID NO: 18, (b) einer Sequenz mit mehr als 90 % Sequenzidentität zu HRV14 VP4 SEQ ID NO: 18, (c) SEQ ID NO: 18 mit von 1-25 Aminosäureadditionen oder -deletionen an einem der Enden und (d) SEQ ID NO: 18 mit 1-50 Aminosäuresubstitutionen oder -deletionen innerhalb der Sequenz.

11. Immunogene Zusammensetzung, die das VLP gemäß irgendeinem der vorherigen Ansprüche und ein(en) pharmazeutisch akzeptables/akzeptablen Verdünnungsmittel, Hilfsstoff oder Träger umfasst.

12. VLP gemäß irgendeinem der Ansprüche 1 bis 10 oder immunogene Zusammensetzung gemäß Anspruch 11 zur Verwendung in einem Verfahren zur Induktion einer Immunantwort gegen humanes Rhinovirus in einem Menschen.

13. VLP gemäß irgendeinem der Ansprüche 1 bis 10 oder immunogene Zusammensetzung gemäß Anspruch 11, wobei das VLP durch ein Verfahren zur Herstellung rhinoviraler VLPs hergestellt ist, umfassend die Schritte:
a. des Herstellens von Konstrukten, die für die Bildung eines VLPs benötigte Kapsidproteine kodieren, wobei jedes der Kapsidproteine als ein Fusionsprotein mit einem Chaperon-Protein kodiert ist,
b. des Co-Exprimierens der Konstrukte in derselben Wirtszelle oder des Exprimierens von einem oder mehreren der Konstrukte in separaten Wirtszellen, und
c. des Lysierens der Wirtszellen und, im Falle einer separaten Expression, des Zusammenbringens aller für ein VLP benötigten Kapsidproteine.

14. VLP oder immunogene Zusammensetzung gemäß Anspruch 13, wobei das Verfahren weiter Schritt d. des Entfernens des Chaperon-Proteins umfasst.

15. VLP oder immunogene Zusammensetzung gemäß Anspruch 13, wobei das Chaperon-Protein ein kleiner Ubiquitin-verwandter Modifikator (Small Ubiquitin-related Modifier, SUMO)-Protein ist.

## Revendications

1. Particule de type virus (VLP), comprenant des protéines de capside de rhinovirus humain VP0, VP1 et VP3, ou, comprenant des protéines de capside de rhinovirus humain VP1, VP2, VP3 et VP4.

2. VLP selon la revendication 1, dans laquelle toutes les protéines de capside proviennent du même sérotype de rhinovirus humain.

3. VLP selon la revendication 1, dans laquelle les protéines de capside proviennent de deux ou plus de deux sérotypes différents de rhinovirus humain.

4. VLP selon l'une quelconque des revendications précédentes, dans laquelle une ou plusieurs des protéines de capside comprennent au moins une modification qui augmente la capacité de la VLP à induire une réponse immunitaire à réaction croisée contre de multiples sérotypes de rhinovirus humain, par comparaison à une VLP dans laquelle ladite modification n'est pas présente, ladite modification consistant en une insertion d'au moins un peptide à partir d'une protéine de capside rhino viral, ledit peptide étant capable d'induire une réponse immunitaire à réaction croisée contre deux ou plus de deux sérotypes de rhinovirus humain.

5. VLP selon l'une quelconque des revendications précédentes, dans laquelle la VLP comprend au moins une modification qui entraîne une atténuation d'un épitope immunodominant.

6. VLP selon l'une quelconque des revendications précédentes, dans laquelle la protéine VP0 a une séquence sélectionnée parmi le groupe constitué de (a) la SEQ ID NO: 14; (b) une séquence ayant plus de 90 % d'identité de séquence avec la SEQ ID NO : 14, (c) la SEQ ID NO : 14 ayant de 1 à 25 additions ou délétions d'acides aminés à l'une ou l'autre extrémité, et (d) la SEQ ID NO : 14 ayant de 1 à 50 substitutions ou délétions d'acides aminés dans la séquence.

7. VLP selon l'une quelconque des revendications précédentes, dans laquelle la protéine VP1 a une séquence sélectionnée parmi le groupe constitué de (a) la SEQ ID NO: 15, (b) une séquence ayant plus de 90 % d'identité de séquence avec la SEQ ID NO : 15, (c) la SEQ ID NO : 15 ayant de 1 à 25 additions ou délétions d'acides aminés à l'une ou l'autre extrémité, et (d) la SEQ ID NO : 15 ayant de 1 à 50 substitutions ou délétions d'acides aminés dans la séquence.

8. VLP selon l'une quelconque des revendications précédentes, dans laquelle la protéine VP2 a une séquence sélectionnée parmi le groupe constitué de (a) la SEQ ID NO: 16, (b) une séquence ayant plus de 90 % d'identité de séquence avec la SEQ ID NO : 16, (c) la SEQ ID NO : 16 ayant de 1 à 25 additions ou délétions d'acides aminés à l'une ou l'autre extrémité, et (d) la SEQ ID NO : 16 ayant de 1 à 50 substitutions ou délétions d'acides aminés dans la séquence.

9. VLP selon l'une quelconque des revendications précédentes, dans laquelle la protéine VP3 a une séquence sélectionnée parmi le groupe constitué de (a) la SEQ ID NO: 17, (b) une séquence ayant plus de 90 % d'identité de séquence avec la SEQ ID NO : 17, (c) la SEQ ID NO : 17 ayant de 1 à 25 additions ou délétions d'acides aminés à l'une ou l'autre extrémité, et (d) la SEQ ID NO : 17 ayant de 1 à 50 substitutions ou délétions d'acides aminés dans la séquence.

10. VLP selon l'une quelconque des revendications précédentes, dans laquelle la protéine VP4 a une séquence sélectionnée parmi le groupe constitué de (a) la SEQ ID NO : 18, (b) une séquence ayant plus de 90 % d'identité de séquence avec la HRV4 VP4 SEQ ID NO : 18, (c) la SEQ ID NO : 18 ayant de 1 à 25 additions ou délétions d'acides aminés à l'une ou l'autre extrémité, et (d) la SEQ ID NO : 18 ayant de 1 à 50 substitutions ou délétions d'acides aminés dans la séquence.

11. Composition immunogène comprenant la VLP selon l'une quelconque des revendications précédentes et un diluant, excipient ou support pharmaceutiquement acceptable.

12. VLP selon l'une quelconque des revendications 1 à 10 ou composition immunogène selon la revendication 11 à utiliser dans un procédé d'induction d'une réponse immunitaire chez un être humain contre un rhinovirus humain.

13. VLP selon l'une quelconque des revendications 1 à 10 ou composition immunogène selon la revendication 11, dans laquelle la VLP est produite par un procédé de production de VLP rhinovirales comprenant les étapes consistant à :
a. produire des constructions codant pour des protéines de capside nécessaires à la formation d'une VLP, chacune des protéines de capside étant codée sous la forme d'une protéine de fusion avec une protéine chaperon,
b. co-exprimer lesdites constructions dans la même cellule hôte ou exprimer une ou plusieurs desdites constructions dans des cellules hôtes séparées, et,
c. lyser lesdites cellules hôtes et rassembler, en cas d'expression séparée, toutes les protéines de capside nécessaires à une VLP.

14. VLP ou composition immunogène selon la revendication 13, dans laquelle le procédé comprend en outre l'étape d. consistant à retirer ladite protéine chaperon.

15. VLP ou composition immunogène selon la revendication 13, dans laquelle la protéine chaperon est une petite protéine modificatrice de type ubiquitine (SUMO).
